(19) Europäisches Patentamt
European Patent Office
Office européen des brevets

(11) Publication number: **0 168 574 B1**

# (12) EUROPEAN PATENT SPECIFICATION

(45) Date of publication of patent specification: **04.03.92** (51) Int. Cl.[5]: **A61K 7/06**

(21) Application number: **85105654.9**

(22) Date of filing: **08.05.85**

## (54) Hair dressing composition.

(30) Priority: **18.05.84 JP 99820/84**

(43) Date of publication of application:
**22.01.86 Bulletin 86/04**

(45) Publication of the grant of the patent:
**04.03.92 Bulletin 92/10**

(84) Designated Contracting States:
**AT CH DE GB LI NL**

(56) References cited:

**CHEMICAL ABSTRACTS, vol. 97, no. 22, 29th November 1982, page 371, abstract no. 188096r, Columbus, Ohio, US; & FR-A-2 498 076 (KAO SOAP CO., LTD) 23-07-1982**

**CHEMICAL ABSTRACTS, vol. 94, no. 12, March 1981, page 392, abstract no. 90042h, Columbus, Ohio, US; & JP-A-80 79 314 (NISSHIN OIL MILLS, LTD) 14-06-1980**

(73) Proprietor: **Kao Corporation**
**14-10, Nihonbashi Kayabacho 1-chome**
**Chuo-Ku Tokyo 103(JP)**

(72) Inventor: **Ando, Hiroshi**
**1-13-11, Koyadai**
**Funabashi-shi Chiba-ken(JP)**

(74) Representative: **Wächtershäuser, Günter, Dr.**
**Tal 29**
**W-8000 München 2(DE)**

EP 0 168 574 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

## BACKGROUND OF THE INVENTION

### i) Field of the Invention:

This invention relates to hair dressing compositions and more particularly, to hair dressing compositions which comprise polyoxyalkylene compounds and a specific type of monoglyceryl ether and which are soft and non-sticky to the touch and have adequate dressing ability.

### ii) Description of the Prior Art:

In order to assist dressing of human hair in a desired style and keep the dressed hair over a long term, it is the common practice to use a variety of hair dressings such as hair liquids, hair creams, set lotions, hair sprays, blow lotions and the like.

These hair dressings comprise various oils and resins. If oils are used in large amounts, a certain extent of dressing ability is obtained, but the resulting hair dressing becomes oily to the touch. On the other hand, resins are disadvantageous in that they have the good dressing ability but impart stiffness to the dressed hair.

Polyoxyalkylene compounds such as polyoxypropylene butyl ether show less tendencies toward an oily feel and stiffness after use, and are thus one of dressing ingredients which are favorably used in recent years. However, even these compounds are not satisfactory in order to impart a natural and soft feel to the hair. More particularly, such polyoxyalkylene compounds have generally an increasing dressing ability as the degree of polymerization increases. However, the sticky feel also increases. On the other hand, when the degree of polymerization decreases, the stickiness decreases with an attendant disadvantage that the dressing ability also decreases. Accordingly, a certain degree of polymerization is selected in order to hold a balance between the dressing ability and the stickiness. However, there is a demand of increasing the dressing ability without increasing the stickiness. FR-A- 2 498 076 discloses a hair cosmetic composition comprising a polyoxyalkylene compound (A) and an α-monostearyl glyceryl ether (B) in a ratio A:B = 1:8.3

## SUMMARY OF THE INVENTION

We have made intensive studies in order to overcome the disadvantages involved in polyoxyalkylene compounds and, as a result, have found that if a specific type of monoglyceryl ether is used in combination with the polyoxyalkylene compounds in a selected ratio range, the dressing ability increases while decreasing the sticky feel.

According to the present invention, there is provided a hair dressing composition characterized by comprising (A) a polyoxyalkylene compound obtained by subjecting a monohydric or polyhydric alcohol to addition polymerization with an alkylene oxide, and (B) an alpha-mono(methyl-branched alkyl) glyceryl ether of the following general formula (I)

$$R\text{-}OCH_2CH(OH)CH_2OH \qquad (I)$$

in which R represents a methyl-branched saturated hydrocarbon group having from 12 to 24 carbon atoms, said ether being used, on the weight basis, in an amount of 0.01 to 1 time the amount of the compound (A).

## DETAILED DESCRIPTION OF THE INVENTION AND PREFERRED EMBODIMENTS

The polyoxyalkylene compounds, which are (A) ingredient of the hair dressing composition of the invention, are, for example, compounds which are obtained by subjecting aliphatic monohydric alcohols or polyhydric alcohols to addition polymerization with an alkylene oxide. Typical aliphatic monohydric alcohols include methanol, ethanol, butanol, octanol or lauryl alcohol, and typical polyhydric alcohols include propylene glycol, glycerine, 2-ethyl-2-hydroxymethyl-1,3-propanediol, mannitol or sorbitol. Alkylene oxides may be propylene oxide or a mixture of propylene oxide and ethylene oxide, of which propylene oxide is preferred. When ethylene oxide and propylene oxide are used in combination, the moles of added ethylene oxides should be below 1/3 of the total addition moles. Ethylene oxide should preferably be added in blocks. The number of total addition moles of an alkylene oxide is generally in the range of from 2 to 100, preferably from 6 to 100 moles, and most preferably from 8 to 50 moles.

Preferable polyoxyalkylene compounds are compounds of the following general formulae (III) through (VI).

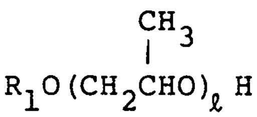

(III)

in which $R_1$ represents an alkyl group having from 1 to 22 carbon atoms, and ℓ is the number of average moles of added propylene oxide ranging from 10 to 100.

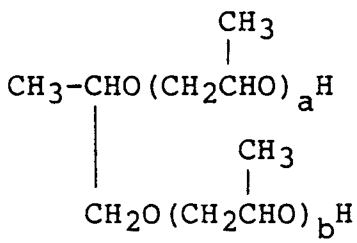

(IV)

in which (a + b) indicating the number of average moles of added propylene oxide is from 2 to 100,

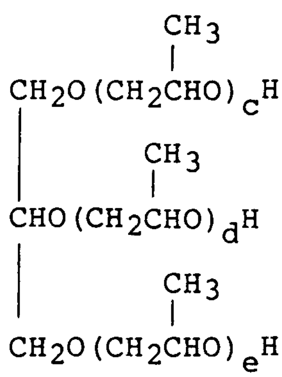

(V)

in which (c + d + e) indicating the number of average moles of added propylene oxide is from 3 to 100,

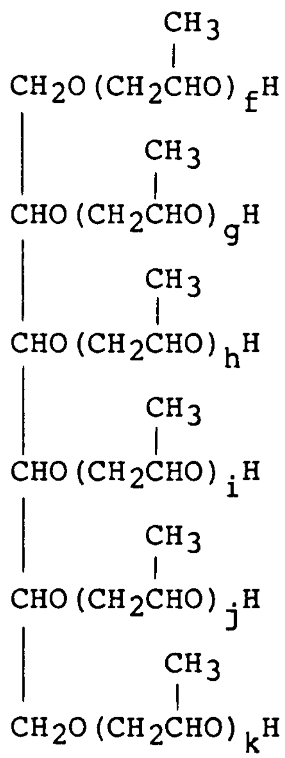

(VI)

in which (f + g + h + i + j + k) indicating the number of average moles of added propylene oxide is from 2 to 100.

The alpha-mono(methyl-branched alkyl) glyceryl ether of the formula (I), which is (B) ingredient of the hair dressing composition of the invention, is preferred to have, as R in the formula, a group of the following formula (II)

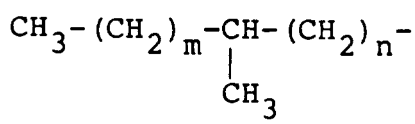

(II)

in which m is an integer of from 2 to 14, and n is an integer of from 3 to 11 provided that m + n is in the range of from 9 to 21, preferably from 11 to 17.

These alpha-mono(methyl-branched alkyl) glyceryl ethers are obtained, for example, by reacting an alcohol of the general formula (VII)

ROH (VII)

in which R has the same meaning as defined before, with a halogenating reagent to obtain a corresponding alkyl halide (VIII), further reacting the alkyl halide with a glycerol alkali metal alcoholate whose hydroxyl groups at the 2 and 3 positions are protected, thereby obtaining a 1-alkyl glyceryl ether (IX) in which the hydroxyl groups at the 2 and 3 positions are protected, and finally subjecting the ether to hydrolysis. The sequence of the above reactions is summarized as follows.

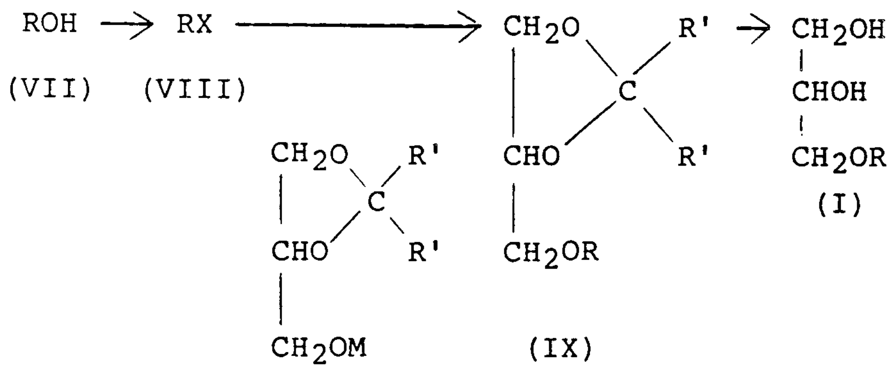

(VII) (VIII) (IX) (I)

in which R' represents a lower alkyl group or a phenyl group, M represents an alkali metal, and R has the same meaning as defined before.

Alternatively, the alpha-mono(methyl-branched alkyl) glyceryl ether of the formula (I) may be prepared by a procedure which comprises subjecting an epihalohydrin to reaction with the alcohol (VII) to obtain an alkyl halohydrin ether (X), ring-closing the ether to obtain an alkyl glycidyl ether (XI), and hydrolyzing the ether (XI). These reactions are shown below.

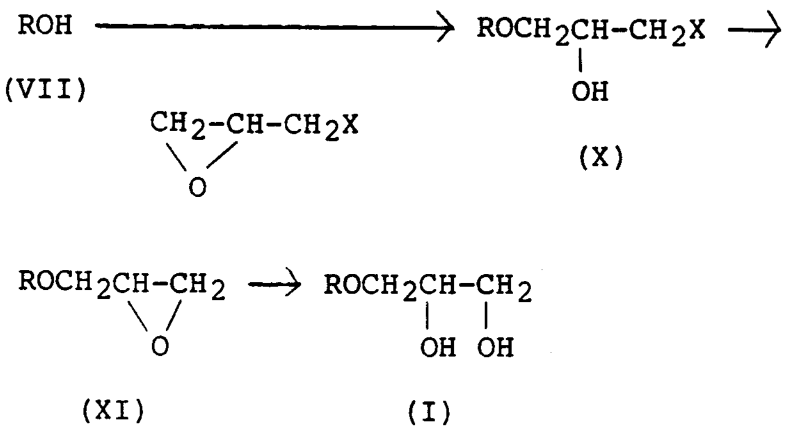

(VII) (X) (XI) (I)

in which X represents a halogen atom, and R has the same meaning as defined before.

The hair dressing composition of the present invention should comprise the alpha-mono(methyl-branched alkyl) glyceryl ether as (B) ingredient in an amount, on the weight basis, of 0.01 to 1.0 time, preferably 0.05 to 0.5 times, the amount of the polyoxyalkylene compound used as (A) ingredient. Less amounts are unfavorable because the resulting dressing has not good hair style-retentivity and cannot acquire a soft and stickiness-free feel. Larger amounts result in a lowering of the hair style retentivity. (A) ingredient should preferably be contained in an amount of from 0.1 to 40 wt% of the total composition though depending on the type of hair dressing.

The hair dressing composition of the invention may be prepared in any forms such as, for example, hair liquids, hair creams, set lotions and hair sprays, by mixing the essential ingredients (A) and (B) and, if necessary, arbitrary ingredients described hereinafter with solvents such as water, lower alcohols such as ethanol and isopropanol, mixed solvents of water and the lower alcohols preferably 10 to 60% by a usual manner. Especially, when aerosol is prepared, there are preferably used chloroalkane propellants such as, for example, trichloromonofluoromethane, dichlorodifluoromethane and dichlorotetrafluoroethane and the like, liquefied petroleum gases, dimethyl ether, or mixtures thereof. These propellants are preferably packed so that the internal pressure in an aerosol can is in the range of 1.96 to 3.92 bar (2.0 to 4.0 $kg/cm^2G$). When the dressing is in the form of a misty aerosol, the propellant should preferably be contained in an amount of from 50 to 80% or more. With a hair set lotion composition in the form of an aerosol foam, the propellant is preferably contained in an amount of from 10 to 30% or less.

Aside from the essential ingredients, the hair dressing composition of the invention comprises various ingredients in amounts not impeding the effect of the invention. Examples of such ingredients are cosmetic

oils including glycerides such as castor oil, cacao oil, mink oil, avogado oil and olive oil; waxes such as beeswax, sperm oil, lanolin and carnauba wax; alcohols such as cetyl alcohol, oleyl alcohol, lauryl alcohol, stearyl alcohol, propylene glycol, polypropylene glycol and glycerine; esters such as isopropyl myristate, hexyl laurate, cetyl lactate, propylene glycol monostearate, oleyl oleate, hexadecyl 2-ethylhexanate and octyldodecyl myristate; and silicone derivatives such as dimethylpolysiloxane, methylphenylpolysiloxane, polyether-modified silicone oils, epoxy-modified silicone oils, amino-modified silicone oils and alkyl-modified silicone oils. Emulsifiers for emulsification and stabilization may further be added. In order to enhance the commercial value, perfumes or colorants may be added. In addition, preservatives or antioxidants for preventing deterioration of dressing compositions with time may be added.

The present invention is more particularly described by way of examples, which should not be construed as limiting the present invention. In examples, hair dressing compositions were evaluated according to the following procedures.

(1) Hair Dressing Ability

A tuft of hair, weighing 4 g, was wetted with water, applied with 1 ml of a hair dressing, combed, and dried with hot air for 30 minutes, followed by evaluating a degree of dishevelling of the hair tuft.

O: No dishevelling of the hair tuft with very good hair dressing ability.

△: Slight degree of dishevelling of the tuft with fair dressing ability.

X: Great degree of dishevelling of the tuft with poor dressing ability.

(2) Unstickiness and Softness

(1) The hair tuft treated in the same manner as in (1) was organoleptically assessed by 10 panel members according to the following standard.

The assessment is shown as the average value of evaluation points of the panel members.

| Evaluation point | |
|---|---|
| +2 | Very good |
| +1 | Fairly good |
| 0 | Moderate |
| -1 | Rather poor |
| -2 | Very poor |

**Example 1**

Hair dressings of the following formulations were prepared to evaluate the dressing ability, unstickiness, and softness. The results are shown in Table 1.

Formulation:

| | |
|---|---|
| Polyoxypropylene glyceryl ether (in formula (V), c + d + e = 15) | 0 or 2.0 (%) |
| Alpha-mono(isostearyl) glyceryl ether (in formula II, m = 7, n = 8) | see Table 1 |
| 95 v/v% ethanol | 50.0 |
| Water | balance |
| | ------- |
| | 100.0 |

Table 1

| Amount (%) | | Evaluation | | |
|---|---|---|---|---|
| Polyoxypropylene glyceryl ether | Alpha-mono (isostearyl) glyceryl ether | Dressing ability | Unstickiness | Softness |
| **Products of Invention:** | | | | |
| 2.0 | 0.02 | O | +1.1 | +1.0 |
| 2.0 | 0.40 | O | +1.5 | +1.8 |
| 2.0 | 1.00 | O | +1.8 | +1.8 |
| **Comparative Products** | | | | |
| 2.0 | 0 | △ | -1.2 | -1.4 |
| 2.0 | 3.0 | △ | -0.6 | +0.2 |
| - | 3.0 | X | +0.3 | -0.2 |

**Example 2**

Hair dressings of the following formulations were prepared to evaluate the dressing ability, unstickiness and softness, the results are shown in Table 2.

Formulation:

Polyoxypropylene compound 4.0(%)

| | |
|---|---|
| Alpha-mono(isostearyl) glyceryl ether (in formula (II), m = 7, n = 8) | 0 or 1.0 |
| Polyoxyethylene (20) lauryl ether | 0.5 |
| Perfume | 0.2 |
| 95 v/v% ethanol | 50.0 |
| Water | balance |
| | ------- |
| | 100.0 |

Table 2

| | Product of Invention | | | | Product for Comparison | | | |
|---|---|---|---|---|---|---|---|---|
| Polyoxypropylene butyl ether (in formula (III), $\ell$ = 40) | 4.0 | | | | 4.0 | | | |
| Polyoxypropylene propylene glycol ether (in formula (IV), a + b = 16) | | 4.0 | | | | 4.0 | | |
| Polyoxypropylene glyceryl ether (in formula (V), c + d + e = 50) | | | 4.0 | | | | 4.0 | |
| Polyoxypropylene sorbitol ether (in formula (VI), f + g + i + j + k = 10) | | | | 4.0 | | | | 4.0 |
| Alpha-mono(isostearyl) glyceryl ether (in formula (II), m = 7, n = 8) | 1.0 | 1.0 | 1.0 | 1.0 | | | | |
| Dressing ability | O | O | O | O | △ | △ | △ | △ |
| Unstickiness | +1.5 | +1.2 | +1.3 | +1.6 | -1.3 | -0.8 | -1.1 | -1.5 |
| Softness | +1.3 | +1.6 | +1.6 | +1.8 | -0.8 | -1.0 | -1.0 | -1.8 |

## Example 3

Hair dressings of the following formulations were prepared to evaluate the dressing ability, unstickiness and softness. The results are shown in Table 3.

Formulation:

| | |
|---|---|
| Polyoxypropylene sorbitol ether (in formula (VI), f + g + h + i + j + k = 10) | 2.0(%) |
| Alpha-mono(isostearyl) glyceryl ether or comparative compound | 0.5 |
| Perfume | 0.3 |
| 95 v/v% ethanol | 50.0 |
| Water | balance |
| | 100.0 |

Table 3

| Compound | Evaluation | | |
|---|---|---|---|
| | Dressing ability | Unsticki-ness | Soft-ness |
| **Product of Invention:** | | | |
| Alpha-mono(isostearyl) glyceryl ether (in formula (II), m = 7, n = 8) | O | +1.7 | +1.5 |
| **Products for Comparison** | | | |
| Polyoxyethylene(10) stearyl ether | X | -0.3 | -0.8 |
| 2-Hexyldecanol | X | -0.4 | -0.1 |
| Isostearic acid | △ | -1.0 | -0.2 |

**Example 4**

Hair dressings of the following formulations were prepared to evaluate the dressing ability, unstickiness and softness. The results are shown in Table 4.

Formulation:

| | |
|---|---|
| Polyoxypropylene sorbitol ether | 0 or 15.0(%) |
| Alpha-mono(isostearyl) glyceryl ether | 0 or 6.0 |
| Softhanol® 90 | 1.0 |
| Perfume | 0.3 |
| 95 v/v% ethanol | 50.0 |
| Water | balance |
| | 100.0 |

Table 4

| Amount (%) | | Evaluation | | |
|---|---|---|---|---|
| Polyoxypropylene sorbitol ether (in formula VI, f + g + h + i + j + k = 50) | Alpha-mono(isostearyl) glyceryl ether (in formula (II), m = 7, n = 8) | Dressing ability | Unsticki-ness | Soft-ness |
| **Product of Invention:** | | | | |
| 15.0 | 6.0 | O | +1.1 | +1.9 |
| **Comparative Products** | | | | |
| - | 6.0 | X | +0.2 | +0.2 |
| 15.0 | - | △ | -1.6 | -1.7 |

## Claims

1. A hair dressing composition characterized by comprising (A) a polyoxyalkylene compound obtained by subjecting a monohydric or polyhydric alcohol to addition polymerization with an alkylene oxide, and (B) an alpha-mono(methyl-branched alkyl) glyceryl ether of the following general formula (I)

$R\text{-}OCH_2CH(OH)CH_2OH$ (I)

in which R represents a methyl-branched saturated hydrocarbon group having from 12 to 24 carbon atoms, said ether being used, on the weight basis, in an amount of 0.01 to 1 time the amount of the compound (A).

**2.** A hair dressing composition according to Claim 1, wherein R in the formula (I) is a group of the following formula (II)

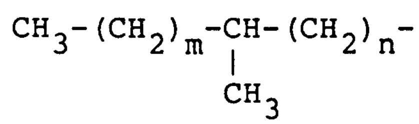

(II)

in which m is an integer of from 2 to 14, and n is an integer of from 3 to 11 provided that m + n is in the range of from 9 to 21.

**3.** A hair dressing composition according to Claim 1, wherein said polyoxyalkylene compound is a compound of the general formula (III)

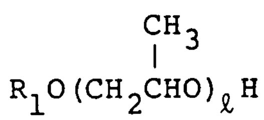

(III)

in which $R_1$ represents an alkyl group having from 1 to 22 carbon atoms, and ℓ is the number of average moles of added propylene oxide ranging from 10 to 100.

**4.** A hair dressing composition according to Claim 1, wherein said polyoxyalkylene compound is a compound of the following formula (IV)

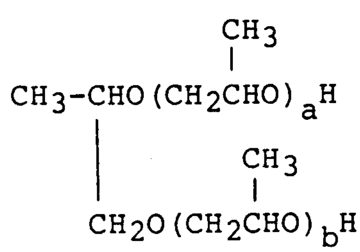

(IV)

in which (a + b) indicating the number of average moles of added propylene oxide is from 2 to 100.

**5.** A hair dressing composition according to Claim 1, wherein said polyoxyalkylene compound is a compound of the general formula (V)

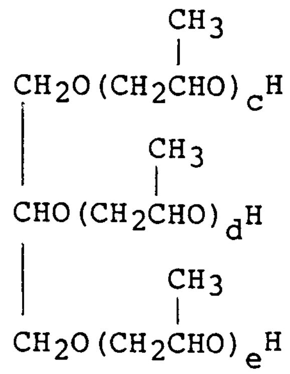

(V)

in which (c + d + e) indicating the number of average moles of added propylene oxide is from 3 to 100.

6. A hair dressing composition according to Claim 1, wherein said polyalkylene oxide is a compound of the general formula (VI)

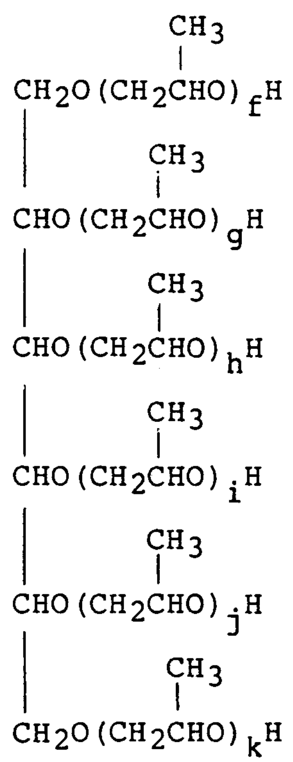

(VI)

in which (f + g + h + i + j + k) indicating the number of average moles of added propylene oxide is from 2 to 100.

7. A hair dressing composition according to any one of the preceding claims 1 to 6, wherein the content of said polyoxyalkylene compound is in the range of from 0.1 to 40 wt% of the total composition.

**Revendications**

1. Composition de coiffage des cheveux, caractérisée par le fait qu'elle comprend :
   (A) un composé de polyoxyalkylène que l'on a obtenu en soumettant un mono- ou polyalcool à une polymérisation par addition avec un oxyde d'alkylène ; et
   (B) un alpha-mono(alkyl à ramification méthyle) glycéryl éther de formule générale (I) suivante :

R-$OCH_2CH(OH)CH_2OH$ (I)

dans laquelle R représente un groupe hydrocarboné saturé à ramification méthyle, ayant de 12 à 24 atomes de carbone,

ledit éther étant utilisé, sur une base en poids,en une quantité de 0,01 à 1 fois la quantité du composé (A).

**2.** Composition de coiffage des cheveux selon la revendication 1, dans laquelle R dans la formule (I) est un groupe de formule (II) suivante :

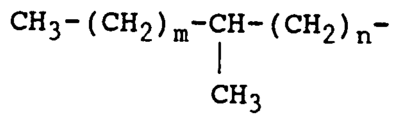

(II)

dans laquelle m est un nombre entier de 2 à 14, et n est un nombre entier de 3 à 11, à la condition que m + n se situe dans la plage de 9 à 21.

**3.** Composition de coiffage des cheveux selon la revendication 1, dans laquelle ledit composé de polyoxyalkylène est un composé de formule générale (III) :

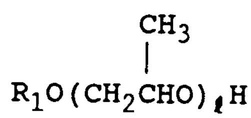

(III)

dans laquelle :

- $R_1$ représente un groupe alkyle ayant de 1 à 22 atomes de carbone ; et
- $\ell$ est le nombre de moles moyennes d'oxyde de propylène ajouté se situant dans la plage de 10 à 100.

**4.** Composition de coiffage des cheveux selon la revendication 1, dans laquelle ledit composé de polyoxyalkylène est un composé de formule générale (IV) suivante :

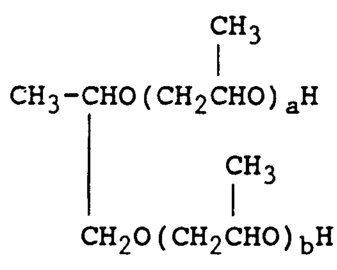

(IV)

dans laquelle (a + b), indiquant le nombre de moles moyennes d'oxyde de propylène ajouté, est de 2 à 100.

**5.** Composition de coiffage des cheveux selon la revendication 1, dans laquelle ledit composé de polyoxyalkylène est un composé de formule générale (V) :

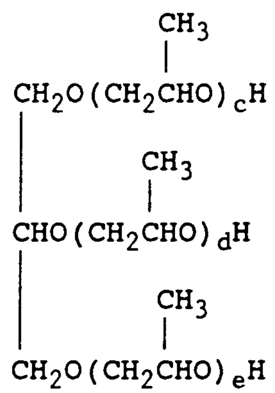

(V)

dans laquelle (c + d + e), indiquant le nombre de moles moyennes d'oxyde de propylène ajouté, est de 3 à 100.

6. Composition de coiffage des cheveux selon la revendication 1, dans laquelle ledit composé de polyalkylène oxyde est un composé de formule générale (VI) :

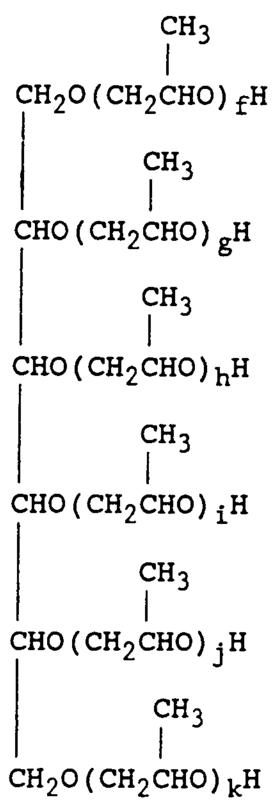

(VI)

dans laquelle (f + g + h + i + j + k), indiquant le nombre de moles moyennes d'oxyde de propylène ajouté, est de 2 à 100.

7. Composition de coiffage des cheveux selon l'une quelconque des revendications 1 à 6 précédentes, dans laquelle la teneur dudit composé de polyoxyalkylène se situe dans la plage de 0,1 à 40% en poids de la composition totale.

## Patentansprüche

1. Haarpflegemittel, gekennzeichnet durch einen Gehalt an (A) einer Polyoxyalkylenverbindung, die

erhalten wurde, indem man einen einwertigen oder mehrwertigen Alkohol der Additionspolymerisation mit einem Alkylenoxid unterwirft, und (B) einem α-Mono(methyl-verzweigt.-alkyl)glyceryl-ether der folgenden allgemeinen Formel (I):

$R\text{-}OCH_2CH(OH)CH_2OH$ (I)

in der R für eine Methyl-verzweigte, gesättigte Kohlenwasserstoffgruppe mit 12 bis 24 Kohlenstoffatomen steht, wobei der Ether auf Gewichtsbasis in einer Menge vom 0,01 bis 1-fachen der Menge der Verbindung (A) verwendet wird.

2. Haarpflegemittel gemäß Anspruch 1, wobei R in der Formel (I) eine Gruppe der folgenden Formel (II) ist:

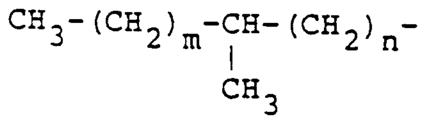

(II)

in der m für eine ganze Zahl von 2 bis 14 steht und n eine ganze Zahl von 3 bis 11 ist, mit der Maßgabe, daß m + n im Bereich von 9 bis 21 liegt.

3. Haarpflegemittel gemäß Anspruch 1, wobei die Polyoxyalkylenverbindung eine Verbindung der allgemeinen Formel (III) ist:

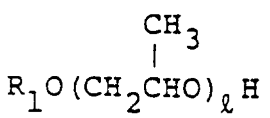

(III)

in der $R_1$ für eine Alkylgruppe mit 1 bis 22 Kohlenstoffatomen steht und ℓ die Zahl der Mol durchschnittlich addiertes Propylenoxid ist und im Bereich von 10 bis 100 liegt.

4. Haarpflegemittel gemäß Anspruch 1, wobei die Polyoxyalkylenverbindung die folgende Formel (IV) aufweist:

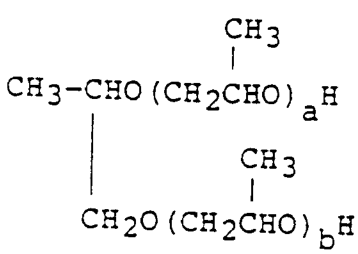

(IV)

in der (a + b), was die Anzahl der Mol durchschnittlich addiertes Propylenoxid anzeigt, von 2 bis 100 beträgt.

5. Haarpflegemittel gemäß Anspruch 1, wobei die Polyoxyalkylenverbindung eine Verbindung der allgemeinen Formel (V) ist:

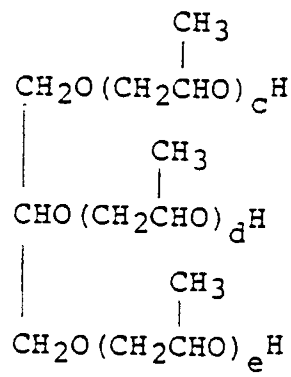

(V)

in der (c + d + e), was die Anzahl der Mol durchschnittlich addiertes Propylenoxid anzeigt, von 3 bis 100 beträgt.

**6.** Haarpflegemittel gemäß Anspruch 1, wobei die Polyoxyalkylenverbindung eine Verbindung der allgemeinen Formel (VI) ist:

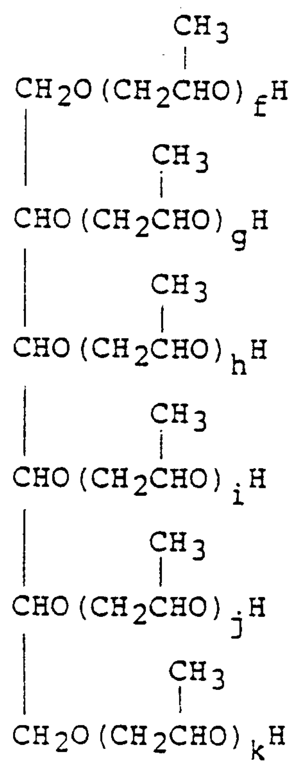

(VI)

in der (f + g + h + i + j + k), was die Anzahl der Mol durchschnittlich addiertes Propylenoxid anzeigt, von 2 bis 100 beträgt.

**7.** Haarpflegemittel gemäß einem der vorstehenden Ansprüche 1 bis 6, wobei der Gehalt der Polyoxyalkylenverbindung im Bereich von 0,1 bis 40 Gewichtsprozent, bezogen auf das Mittel, beträgt.